(19) Europäisches Patentamt
European Patent Office
Office européen des brevets

(11) **EP 1 943 511 B1**

(12) **FASCICULE DE BREVET EUROPEEN**

(45) Date de publication et mention
de la délivrance du brevet:
**18.03.2009 Bulletin 2009/12**

(21) Numéro de dépôt: **06831303.0**

(22) Date de dépôt: **26.10.2006**

(51) Int Cl.:
*G01N 33/28* (2006.01)      *G01N 30/02* (2006.01)
*G01N 30/86* (2006.01)      *C10M 175/02* (2006.01)
*C10M 101/02* (2006.01)

(86) Numéro de dépôt international:
**PCT/FR2006/051111**

(87) Numéro de publication internationale:
**WO 2007/051941 (10.05.2007 Gazette 2007/19)**

(54) **PROCEDE DE DETERMINATION DE LA TENEUR EN GAZOLE DANS UNE HUILE LUBRIFIANTE DE MOTEUR A COMBUSTION**

VERFAHREN ZUR BESTIMMUNG DES DIESELKRAFTSTOFFGEHALTS EINES SCHMIERÖLS FÜR EINEN VERBRENNUNGSMOTOR

METHOD FOR DETERMINING THE CONTENT OF DIESEL FUEL IN A LUBRICATING OIL OF A COMBUSTION ENGINE

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **03.11.2005 FR 0511195**

(43) Date de publication de la demande:
**16.07.2008 Bulletin 2008/29**

(73) Titulaire: **RENAULT S.A.S.**
**92100 Boulogne Billancourt (FR)**

(72) Inventeurs:
• **FITAMEN, Eric**
**F-92260 Fontenay Aux Roses (FR)**
• **TIQUET, Laurent**
**F-28320 Gallardon (FR)**

(74) Mandataire: **Guyon, Rodolphe C.**
**Renault Technocentre**
**Service 0267**
**TCR GRA 236**
**1, Avenue du Golf**
**78288 Guyancourt Cedex (FR)**

(56) Documents cités:
**US-A1- 2003 194 811**

• **ANONYMOUS: "ASTM D3524-04: Standard Test Method for Diesel Fuel Diluent in Used Engine Oils by Gas Chromatography" ASTM, [Online] novembre 2004 (2004-11), XP009067885 American Society for Testing and Materials Extrait de l'Internet: URL:http:// webstore.ansi.org/ansidocstore/ product.asp? sku=ASTM+D3524%2D04> [extrait le 2006-06-14]**
• **ANONYMOUS: "DIN 51380: Determination of readily volatile components in used automotive engine oils by gas chromatography" DIN, [Online] novembre 1990 (1990-11), XP009068152 Beuth Verlag GmbH, Berlin Extrait de l'Internet: URL: http://webstore.ansi.org/ansidocstore/ product.asp?sku=DIN+51380> [extrait le 2006-06-20]**

## Description

L'invention concerne l'analyse d'huiles lubrifiantes, et en particulier la détermination de la teneur en carburant dans l'huile d'un moteur à combustion interne.

Des méthodes d'analyse de la teneur en gazole dans une huile lubrifiante sont connues mais ne donnent pas entièrement satisfaction, notamment en ce qui concerne la fidélité du résultat de l'analyse. En effet, il est difficile de différencier le gazole qui comporte en majeure partie des hydrocarbures saturés et insaturés en $C_6$ à $C_{25}$ et l'huile lubrifiante qui comporte en majeure partie des hydrocarbures saturés et insaturés en $C_{20}$ à $C_{50}$. Une très grande fiabilité des résultats est requise, notamment pour la mise au point de moteurs équipés de filtres à particules.

L'invention vise à résoudre plusieurs de ces inconvénients. L'invention propose ainsi un procédé de détermination de la proportion de gazole dans une huile de lubrification de moteur à combustion, comprenant les étapes suivantes :

- former un mélange contenant un échantillon d'huile de lubrification à analyser et un hydrocarbure en $C_5$ tel qu'un alcane en $C_5$ selon une proportion prédéterminée ;
- injecter du mélange dans l'injecteur d'un chromatographe en phase gazeuse ;
- établir un chromatogramme de l'échantillon à analyser ;
- déterminer un premier paramètre M représentatif de l'aire d'un pic du chromatogramme associé à l'hydrocarbure en $C_5$ tel qu'un alcane en $C_5$;
- déterminer un second paramètre C représentatif de l'aire d'au moins un pic du chromatogramme associé à un hydrocarbure représentatif du gazole ;
- déterminer la proportion T en gazole de l'échantillon à analyser par la formule suivante :

$$ T = \frac{C/(M)-b}{a} $$

avec a et b des constantes définissant l'équation y=ax+b d'une droite d'étalonnage du rapport entre le second et le premier paramètres en fonction de la proportion en gazole.

La méthode standard ASTM D3524-04 décrit un procédé similaire utilisant cependant le n-décane (alcane en $C_{10}$) comme étalon interne.

D'autres caractéristiques et avantages de l'invention ressortiront clairement de la description qui en est faite ci-après, à titre indicatif et nullement limitatif, en référence aux dessins annexés, dans lesquels :

- la figure 1 illustre schématiquement un chromatographe selon l'état de la technique ;

- la figure 2 illustre des exemples de paramètres du chromatographe utilisés dans le cadre de l'invention ;
- la figure 3 illustre un exemple de droite d'étalonnage.

L'invention propose de déterminer la teneur en gazole dans un échantillon d'huile de lubrification d'un moteur à combustion interne. Pour cela, on forme un mélange contenant un échantillon d'huile de lubrification à analyser et un hydrocarbure en $C_5$ tel qu'un alcane en $C_5$ selon une proportion prédéterminée. Un échantillon est injecté dans le chromatographe et un chromatogramme de l'échantillon est réalisé. Un paramètre représentatif de l'aire du pic de l'hydrocarbure en $C_5$ est déterminé, ainsi qu'un paramètre représentatif d'un pic associé à un hydrocarbure représentatif du gazole. A partir de données d'étalonnage de ces paramètres, la teneur en gazole dans l'échantillon est déterminée.

On appelle dans la demande de brevet un hydrocarbure en $C_n$, une famille de composés isomères avec n supérieur à 5.

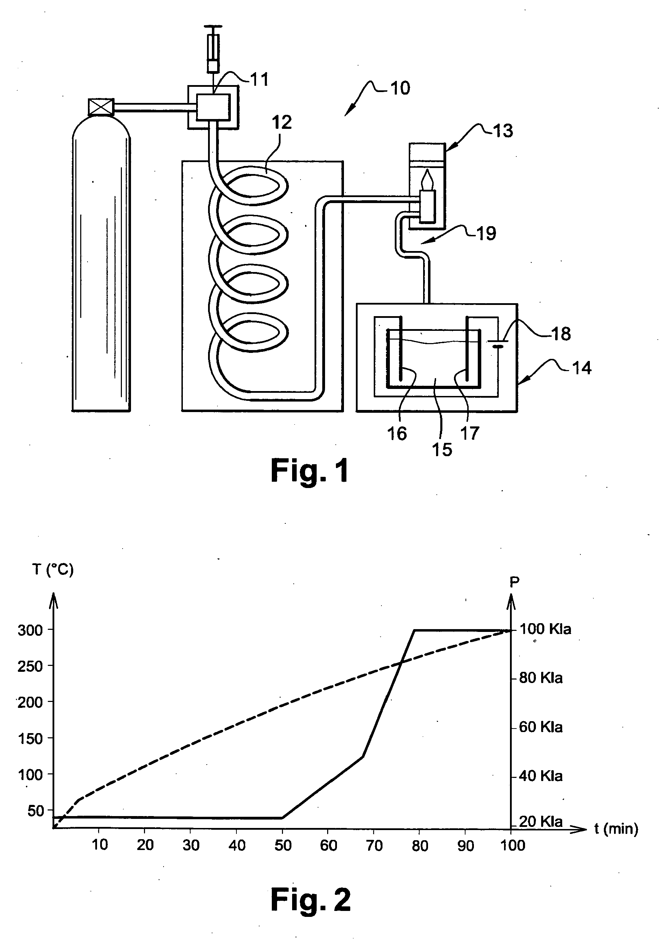

La figure 1 illustre un chromatographe connu de l'état de la technique. Le chromatographe 10 comporte un injecteur 11 pour des produits à analyser, une colonne 12 de séparation des produits à analyser, un détecteur 13 desdits produits et un dispositif 14 de production de gaz de combustion.

La colonne de séparation 12 est traversée en un temps différent par les différents composés des produits à analyser. Le détecteur 13 est par exemple un détecteur à ionisation de flamme connu de l'homme de métier et présente une flamme alimentée par les gaz provenant du dispositif 14. Ce dispositif 14 présente par exemple un réservoir d'eau distillée et un dispositif d'électrolyse 15 contenant de l'eau distillée dans laquelle plongent deux électrodes 16 et 17. Ces électrodes sont alimentées par une source d'énergie électrique 18. De l'hydrogène et de l'oxygène se forment aux électrodes et alimentent le détecteur à ionisation de flamme en oxygène et en hydrogène par un conduit 19. Le détecteur 13 reçoit en outre un gaz de transport des produits dans la colonne de séparation, par exemple de l'hydrogène, de l'hélium ou de l'azote. Le détecteur 13 mesure une intensité générée lors de la combustion d'un composé issu de la colonne 12. L'intensité générée varie en fonction de la présence ou de l'absence d'un composé dans la chambre. Un chromatogramme est généré pour représenter par exemple l'intensité en fonction du temps.

L'invention propose de former un mélange contenant un échantillon d'huile de lubrification à analyser et l'hydrocarbure en $C_5$ tel qu'un alcane en $C_5$ selon des proportions prédéterminées. Le mélange est injecté dans l'injecteur du chromatographe. Un chromatogramme de l'échantillon à analyser est établi. Un premier paramètre M représentatif de l'aire d'un pic associé à l'hydrocarbure en $C_5$ est déterminé. Un second paramètre C représentatif de l'aire d'au moins un pic associé à un hydrocarbure représentatif du gazole est également déterminé. La proportion T en gazole de l'échantillon à analyser est calculée par la formule suivante :

$$T = \frac{C/M - b}{a}$$

a et b étant des constantes définissant la droite d'étalonnage du chromatographe définie généralement par C/M=a.T + b. Les paramètres sont par exemple proportionnels aux amplitudes ou aux aires des pics du chromatogramme.

Le second paramètre peut par exemple être fonction de la hauteur ou de l'aire d'un pic associé à un hydrocarbure appartenant au groupe $C_6$ à $C_{25}$.

On peut envisager de calculer au préalable une approximation de la teneur en gazole en prenant en compte un second paramètre fonction seulement du pic de l'hydrocarbure en $C_{20}$. En fonction de cette approximation, on peut alors déterminer les pics de quels hydrocarbures seront pris en compte dans le paramètre C, pour calculer la teneur en gazole. Le nombre de pics pris en compte peut ainsi être fonction de l'approximation calculée.

Avantageusement, le second paramètre peut être représentatif de l'aire de plusieurs pics associés à des hydrocarbures respectifs appartenant au groupe $C_{20}$ à $C_{25}$.

Afin de tenir compte d'une aire relative des différents composés pour différentes conditions chromatographiques, le premier paramètre est avantageusement corrigé pour ramener la concentration du composé en $C_5$ à une valeur souhaitée. M peut notamment être déterminé par la formule suivante : M= $\alpha$.M0/$[C_5]$

M0 étant l'aire du pic associé à l'hydrocarbure en $C_5$ tel qu'un alcane en $C_5$, $\alpha$ un coefficient de correction vers une concentration de référence et $[C_5]$ la concentration de l'hydrocarbure en $C_5$ dans l'échantillon.

Le mélange formé peut comprendre une proportion prédéterminée de bisulfure de carbone. La fonction du Bisulfure de carbone est de diluer l'ensemble du mélange huile + $C_5$ afin de les mélanger de façon homogène et de disposer d'un milieu liquide et fluide dont la séparation est facilitée. Par ailleurs, le Bisulfure de carbone est avantageusement invisible au détecteur, même en grande quantité. Le Bisulfure de carbone ne perturbe donc pas la détection du gazole et de l'huile.

La droite d'étalonnage est de préférence réalisée au préalable avec le même type de gazole et le même type d'huile que dans l'échantillon à analyser.

Pour la détermination préalable de l'équation de la droite d'étalonnage, les étapes suivantes peuvent être réalisées. On forme plusieurs mélanges étalons comprenant une huile de lubrification et du gazole selon des proportions distinctes prédéfinies, et de l'hydrocarbure en $C_5$ tel qu'un alcane en $C_5$ selon une proportion prédéterminée. La teneur d'un échantillon étalon en gazole sera notée par la suite T0. Pour chaque mélange étalon :

- on injecte le mélange étalon dans l'injecteur d'un chromatographe en phase gazeuse ;
- on établit un chromatogramme du mélange étalon ;
- on détermine un premier paramètre M0 représentatif de l'aire d'un pic du chromatogramme associé à l'hydrocarbure en $C_5$ tel qu'un alcane en $C_5$;
- on détermine un second paramètre C0 représentatif de l'aire d'un pic du chromatogramme associé à un hydrocarbure représentatif du gazole.

A partir des couples de paramètres obtenus pour les différents mélanges obtenus, (pouvant être représentés par des points d'abscisse T0 et d'ordonnée C0/M0), on détermine les constantes a et b. Les constantes a et b sont par exemple obtenues en prenant comme droite d'étalonnage la droite correspondant le mieux aux différents couples formés.

Le chromatogramme peut être établi par un détecteur à ionisation de flamme, un organe de calcul pouvant réaliser des intégrations du chromatogramme afin de calculer l'aire de chaque pic.

On va maintenant détailler un exemple de conditions de mise en oeuvre du procédé selon l'invention.

L'appareillage suivant peut être utilisé :

- un chromatographe en phase gazeuse, présentant de préférence une programmation et une régulation précises de la température et de la pression dans la colonne. Le chromatographe est avantageusement alimenté par de l'hélium, de l'air de qualité industrielle et de l'hydrogène de qualité N55 ;
- une colonne capillaire apolaire commercialisée par la firme Chrompack sous la référence CP Syl 19 cb, présentant une longueur de 10 mètres et un diamètre de 0,53 mm avec une épaisseur de film de 1μm ;
- une précolonne dépourvue de silice de 2 mètres équipée d'une union en verre formant une jonction avec la colonne capillaire ;
- un détecteur à ionisation de flammes (FID) ;
- un dispositif d'intégration sous la forme d'un ordinateur exécutant le logiciel commercialisé sous la référence HPCHEM dans une version ultérieure à A. 04.02 ;
- un injecteur à froid (« on column » en langue anglaise) et présentant avantageusement une régulation de pression et un automate d'injection présentant au moins 8 positions, un passeur à 100 positions étant préconisé ;
- une balance de précision à 0.1mg ;
- une table vibrante destinée à agiter et homogénéiser les échantillons.

Les réactifs suivants peuvent être utilisés :

- du Bisulfure de Carbone (CS$_2$), tel que celui com-

mercialisé par la société Prolabo avec une qualité Normapur pour analyses ;

- du Pentane ($C_5$) présentant une pureté supérieure à 99% ;
- du gazole commercial non additivé ;
- une huile neuve de type ALEA A2/B2 15W40.

Les échantillons étalons suivants peuvent être utilisés. Des mélanges huile neuve/gazole sont préparés et présentent les proportions massiques respectives suivantes en gazole : 1, 2, 3, 4, 5, 6, 7, 8, 10, 12 et 15%.

Pour accroître la précision de l'étalonnage, il est préférable d'utiliser pour ces étalons un gazole et une huile neufs, qui correspondent respectivement au gazole et à l'huile qui ont été utilisés pour obtenir les échantillons à analyser.

Pour chaque échantillon étalon, les étapes suivantes de préparation sont effectuées :

- la masse d'huile correspondant à son titrage massique est prélevée dans un flacon et pesée. Sa masse sera notée $m_1$ ;
- le volume de gazole nécessaire pour obtenir son titrage massique est prélevé et placé dans le flacon. La masse du mélange huile-gazole sera notée $m_2$ ;
- le mélange est secoué vigoureusement pendant au moins 10 minutes avec une table vibrante pour l'homogénéiser.

Les échantillons étalons subissent ensuite le processus décrit ci-dessous pour les échantillons à analyser.

Pour chaque échantillon d'huile à analyser, les étapes de préparation suivantes sont effectuées :

- 1,7 ml de l'échantillon à analyser sont prélevés puis pesés. On notera $m_3$ la masse de cet échantillon. Eventuellement, un nouvel échantillon est prélevé si la masse ne correspond pas à une plage de valeurs attendues ;
- 20 $\mu$l de pentane sont ajoutés à l'échantillon à analyser. Le pentane est stocké et prélevé à température ambiante. On notera $m_4$ la masse du mélange obtenu ;
- le mélange est quasiment immédiatement dilué par un ajout de 5ml de bisulfure de carbone ($CS_2$), le flacon contenant le mélange obtenu est fermé hermétiquement quasiment immédiatement afin de limiter les évaporations du solvant $CS_2$ ;
- le mélange obtenu est agité par une table vibrante pendant approximativement 1 minute pour homogénéiser le mélange. Le mélange alors obtenu est ainsi dilué à 75% volumique dans le $CS_2$ et est prêt à être injecté dans le chromatographe ;
- éventuellement, pour accroître la durée de conservation du mélange obtenu, celui-ci est versé dans une microfiole de 2 ml de façon à garantir un volume mort réduit au minimum. Un bouchon hermétique peut être serti sur l'ouverture de la microfiole. On remplit autant de microfioles que nécessaire pour un mélange donné à analyser ;

- 0,5$\mu$l sont injectés par l'injecteur automatique.

Les volumes et masse ne sont bien entendu donnés qu'à titre indicatif, d'autres valeurs pouvant être utilisées par l'homme de métier.

On va maintenant détailler un exemple d'analyse chromatographique. Durant cette analyse, le gaz vecteur dans la colonne de séparation est l'hélium. Des programmes de température du four et de pression d'hélium du chromatographe peuvent imposer les diagrammes illustrés à la figure 2. La température de l'injecteur est contrôlée pour suivre le programme de température avec 10°C d'avance, selon le principe du suivi de four (oven track en langue anglaise). Le débit du gaz vecteur dans la colonne est conditionné par celui imposé par le programme de pression d'hélium. Le chromatographe fonctionne en régulation de pression.

Pour le chromatographe utilisé par le demandeur durant ses essais, le débit d'hydrogène dans le détecteur était approximativement égal à 30ml/min et le débit d'air dans ce détecteur était égal à 400ml/min à 10ml/min près.

Pour l'intégration des pics des différents composés, les périodes suivantes ont été utilisées dans le cycle de chromatographie :

- l'intégration de la partie étalon Pentane a été effectuée entre t=22secondes et t=33 secondes ;
- l'intégration de la partie Bisulfure de Carbone ($CS_2$) a été effectuée en excluant $C_{20}$, entre t=33 secondes et t=37 minutes et 40 secondes. Les composés entre $C_5$ et $C_{20}$ exclus appartiennent au gazole ;
- l'intégration entre les composés de la partie gazole $C_{20}$ et $C_{22}$ exclus a été effectuée entre t=37 minutes et 40 secondes et t=44 minutes et 15 secondes ;
- l'intégration entre les composés de la partie gazole $C_{22}$ et $C_{24}$ exclus a été effectuée entre t=44 minutes et 15 secondes et t=51 minutes et 15 secondes ;
- l'intégration entre les composés de la partie gazole $C_{24}$ et $C_{25}$ exclus a été effectuée entre t=51 minutes et 20 secondes et t=54 minutes et 20 secondes ;
- l'intégration de la partie huile à partir de $C_{25}$ a été effectuée entre t=54 minutes et 20 secondes et t=97 minutes.

Avantageusement, les temps d'intégration des différents composés sont ajustés en fonction du vieillissement de la colonne.

Les chromatogrammes obtenus sont analysés pour extraire les aires des pics associés aux différents composés. Soit A0 l'aire extraite pour l'étalon pentane, A1 étant l'aire associée au Bisulfure de Carbone, A2 étant l'aire associée jusqu'à $C_{20}$ exclus, A3 étant l'aire associée jusqu'à C22 exclus, A4 étant l'aire associée jusqu'à C24 exclus et A5 étant l'aire associée jusqu'à C25 exclus.

Avantageusement, on ramène la concentration en étalon pentane à une valeur imposée. On pourra ainsi calculer une aire relative des différents composés (ou famille de composés), quelles que soient les conditions chromatographiques.

L'aire corrigée A0' est calculée comme suit :

La masse de Pentane vaut $m_4$-$m_3$. La concentration en Pentane vaut alors $[C_5]=(m_4$-$m_3)/m_4$. Soit $[C_i]$ la concentration imposée (par exemple 0.0066). Ainsi : A0'=A0*$[C_i]$/$[C_5]$

L'établissement de la courbe d'étalonnage est réalisé de la façon suivante :

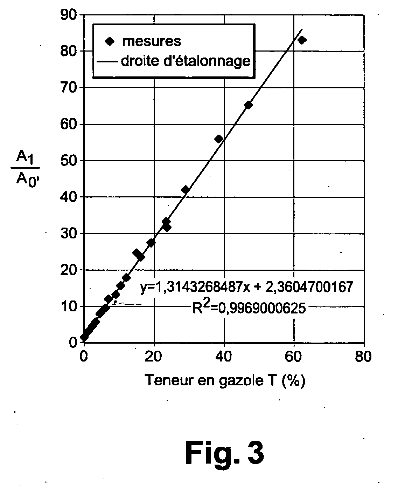

- la teneur réelle pesée en gazole vaut g=$(m_2$-$m_1)/m_2$ ;
- pour chaque aire Ai, avec i compris entre 1 et 4, on trace la droite correspondant approximativement à la fonction $f(g) = (\sum_{k=1}^{i} Ak)/A0'$, par exemple par la méthode des moindres carrés. En exprimant l'équation de la droite sous la forme y=$a_i$x + $b_i$, on détermine les valeurs de $a_i$ et $b_i$. 4 droites d'étalonnage sont ainsi obtenues à partir des mêmes étalons. Un exemple de points et d'une courbe d'étalonnage sont illustrés à la figure 3.

Pour un échantillon à analyser, on réalise de façon similaire les intégrations pour déterminer les aires A0', A1, A2, A3 et A4. Pour i compris entre 1 et 4, on calcule ensuite les rapports $\left(\sum_{k=1}^{i} Ak\right)/A0'$

A partir des rapports obtenus et des droites d'étalonnage, on détermine la teneur en gazole par la formule suivante :

$$\left(\left(\left(\sum_{k=1}^{i} Ak\right)/A0'\right)-b_i\right)/a_i$$

Si le lubrifiant est inconnu, seul le calcul de teneur en gazole pour i=1 est pris en compte.

Pour certaines huiles peu superposées avec les constituants lourds du gazole, on détermine le résultat à prendre en compte en fonction de la teneur en gazole obtenue pour i=1. Par exemple, si cette valeur est comprise entre 0 et 2%, on prend en compte la valeur obtenue pour i=1 ; si cette valeur est comprise entre 2 et 10%, on prend en compte la valeur obtenue pour i=2 ; si cette valeur est comprise entre 10 et 20%, on prend en compte la valeur pour i=3 ; si cette valeur est supérieure à 20%, on prend en compte la valeur pour i=4. On tient alors compte de la co-élution du gazole.

Certaines huiles pouvant présenter une structure particulière (par exemple lors de la présence de C16 dans le mélange de base), une intégration particulière peut être requise. Une telle intégration peut notamment exclure l'aire du pic correspondant au composé en cause et nécessite un étalonnage spécifique.

Avantageusement, le profil de température et de pression du chromatographe sont corrigés périodiquement en fonction d'étalons effectués sur une solution contenant les hydrocarbures en $C_5$, $C_{20}$-$C_{26}$ et $C_{30}$. Par exemple, on pourra utiliser les alcanes en $C_5$, $C_{20}$-$C_{26}$ et $C_{30}$ avec, par exemple, 60mg de chacun de ces composés pour 5 ml de $CS_2$. On tient ainsi compte de l'usure de la colonne et de l'évolution des temps de rétention.

Avantageusement, de nouvelles droites d'étalonnage sont réalisées à chaque nouvelle colonne mise en place dans le chromatographe.

Ce procédé permet de séparer l'huile du gazole en séparant leurs hydrocarbures en $C_n$, n variant de 6 à 50, un hydrocarbure rassemblant une famille de composés saturés et/ou insaturés. Ce procédé n'est pas une séparation de composé par composé mais de famille de composés hydrocarbonés (saturés et/ou insaturés) par famille de composés hydrocarbonés (saturés et/ou insaturés).

**Revendications**

1. Procédé de détermination de la proportion de gazole dans une huile de lubrification de moteur à combustion, **caractérisé en ce qu'**il comprend les étapes suivantes :

- former un mélange contenant un échantillon d'huile de lubrification à analyser et un hydrocarbure en $C_5$ tel qu'un alcane en $C_5$ selon une proportion prédéterminée ;
- injecter du mélange dans l'injecteur (11) d'un chromatographe en phase gazeuse (10) ;
- établir un chromatogramme de l'échantillon à analyser ;
- déterminer un premier paramètre M représentatif de l'aire d'un pic du chromatogramme associé à l'hydrocarbure en $C_5$ tel qu'un alcane en $C_5$;
- déterminer un second paramètre C représentatif de l'aire d'au moins un pic du chromatogramme associé à un hydrocarbure représentatif du gazole ;
- déterminer la proportion T en gazole de l'échantillon à analyser par la formule suivante :

$$T = \frac{C/(M)-b}{a}$$

avec a et b des constantes définissant l'équation y=ax+b d'une droite d'étalonnage du rapport entre le second et le premier paramètres en fonction de la proportion en gazole.

2. Procédé selon la revendication 1, dans lequel le second paramètre C est représentatif de l'aire d'au moins un pic du chromatogramme associé à un hydrocarbure dans le groupe $C_6$ à $C_{25}$.

3. Procédé selon la revendication 2, dans lequel une approximation de la teneur en gazole est calculée en fonction d'un second paramètre C représentatif de l'aire du pic associé à l'hydrocarbure en $C_{20}$ du chromatogramme, puis une teneur en gazole corrigée est calculée en fonction d'un second paramètre C représentatif de l'aire de plusieurs pics associés aux hydrocarbures respectifs choisis dans le groupe $C_{20}$ à $C_{25}$, le nombre de ces pics étant fonction de l'approximation calculée.

4. Procédé selon la revendication 2 ou 3, dans lequel le second paramètre est représentatif de l'aire de plusieurs pics associés à des hydrocarbures respectifs choisis dans le groupe $C_{20}$ à $C_{25}$.

5. Procédé selon l'une quelconque des revendications précédentes, dans lequel le premier paramètre M est établi par la formule suivante :

    M=α.M0/[$C_5$], avec M0 l'aire du pic associé à l'hydrocarbure $C_5$ tel qu'un alcane en $C_5$, α un coefficient de correction vers une concentration de référence et [$C_5$] la concentration de l'hydrocarbure en $C_5$ dans l'échantillon.

6. Procédé selon l'une quelconque des revendications précédentes, **caractérisé en ce que** le mélange formé comprend du Bisulfure de carbone dans une proportion prédéterminée.

7. Procédé selon l'une quelconque des revendications précédentes, comprenant une étape préalable de détermination de l'équation de la droite d'étalonnage consistant à :

    - former plusieurs mélanges étalons comprenant une huile de lubrification et du gazole selon des proportions distinctes prédéfinies, et de l'hydrocarbure en $C_5$ tel qu'un alcane en $C_5$ selon une proportion prédéterminée;
    - pour chaque mélange étalon :

        - injecter le mélange étalon dans l'injecteur (11) d'un chromatographe en phase gazeuse (10) ;
        - établir un chromatogramme du mélange étalon ;

    - déterminer un premier paramètre M0 représentatif de l'aire d'un pic du chromatogramme associé à l'hydrocarbure en $C_5$ tel qu'un alcane en $C_5$;
    - déterminer un second paramètre C0 représentatif de l'aire d'un pic du chromatogramme associé à un hydrocarbure représentatif du gazole ;

    - à partir des couples de premier et seconds paramètres obtenus pour les différents mélanges étalons, déterminer les paramètres a et b de la droite d'étalonnage.

8. Procédé selon l'une quelconque des revendications précédentes, dans lequel chaque chromatogramme est établi par un détecteur à ionisation de flamme (13) et dans lequel un organe de calcul réalise des intégrations du chromatogramme de façon à calculer l'aire de chaque pic du chromatogramme.

**Claims**

1. Method for determining the proportion of diesel fuel in a lubricating oil of a combustion engine, **characterized in that** it comprises the following steps:

    - forming a mixture containing a sample of lubricating oil to be analyzed and a $C_5$ hydrocarbon such as a $C_5$ alkane according to a predetermined proportion;
    - injecting the mixture into the injector (11) of a gas chromatograph (10);
    - establishing a chromatogram of the sample to be analyzed;
    - determining a first parameter M representative of the area of a peak of the chromatogram associated with the $C_5$ hydrocarbon such as a $C_5$ alkane;
    - determining a second parameter C representative of the area of at least one peak of the chromatogram associated with a hydrocarbon representative of the diesel fuel;
    - determining the proportion T of diesel fuel of the sample to be analyzed, by the following formula:

$$T = \frac{C/(M) - b}{a}$$

with a and b being constants defining the equation y = ax+b of a calibration straight line of the ratio between the second and the first parameters as a function of the proportion of diesel fuel.

**2.** Method according to Claim 1, in which the second parameter C is representative of the area of at least one peak of the chromatogram associated with a hydrocarbon in the $C_6$ to $C_{25}$ group.

**3.** Method according to Claim 2, in which an approximation of the content of diesel fuel is calculated as a function of a second parameter C representative of the area of the peakassociated with the $C_{20}$ hydrocarbon of the chromatogram, and then a corrected diesel fuel content is calculated as a function of a second parameter C representative of the area of several peaks associated with the respective hydrocarbons chosen from the $C_{20}$ to $C_{25}$ group, the number of these peaks being a function of the approximation calculated.

**4.** Method according to Claim 2 or 3, in which the second parameter is representative of the area of several peaks associated with respective hydrocarbons chosen from the $C_{20}$ to $C_{25}$ group.

**5.** Method according to any one of the preceding claims, in which the first parameter M is established by the following formula: **M = $\alpha$.M0/[C$_5$],** with M0 being the area of the peakassociated with the $C_5$ hydrocarbon such as a $C_5$ alkane, $\alpha$ being a coefficient of correction to a reference concentration and [$C_5$] being the concentration of the $C_5$ hydrocarbon in the sample.

**6.** Method according to any one of the preceding claims, **characterized in that** the mixture formed comprises carbon disulfide in a predetermined proportion.

**7.** Method according to any one of the preceding claims, comprising a prior step of determining the equation of the calibration straight line, which step consists in:

- forming several standard mixtures comprising a lubricating oil and diesel fuel according to distinct predefined proportions, and $C_5$ hydrocarbon such as a $C_5$ alkane according to a predetermined proportion;
- for each standard mixture:

  - injecting the standard mixture into the injector (11) of a gas chromatograph (10);
  - establishing a chromatogram of the standard mixture;
  - a first parameter M0 representative of the area of a peak of the chromatogram associated with the $C_5$ hydrocarbon such as a $C_5$ alkane determining;
  - determining a second parameter C0 representative of the area of a peak of the chromatogram associated with a hydrocarbon representative of the diesel fuel;

  - based on the pairs of first and second parameters obtained for the various standard mixtures, determining the parameters a and b of the calibration straight line.

**8.** Method according to any one of the preceding claims, in which each chromatogram is established by means of a flame ionization detector (13) and in which a calculation member carries out integrations of the chromatogram so as to calculate the area of each peak of the chromatogram.

**Patentansprüche**

**1.** Verfahren zur Bestimmung des Dieselkraftstoffgehalts eines Schmieröls für einen Verbrennungsmotor, **dadurch gekennzeichnet, daß** man:

- eine Mischung bildet, die eine zu analysierende Schmierölprobe und einen $C_5$-Kohlenwasserstoff wie ein $C_5$-Alkan in einem vorbestimmten Anteil enthält,
- die Mischung in den Injektor (11) eines Gaschromatographen (10) einspritzt;
- ein Chromatogramm der zu analysierenden Probe erstellt;
- einen ersten Parameter M bestimmt, der für die Fläche eines mit dem $C_5$-Kohlenwasserstoff wie einem $C_5$-Alkan assoziierten Peaks des Chromatogramms repräsentativ ist;
- einen zweiten Parameter C bestimmt, der für die Fläche mindestens eines mit einem für Dieselkraftstoff repräsentativen Kohlenwasserstoff assoziierten Peaks des Chromatogramms repräsentativ ist;
- den Dieselkraftstoffanteil T der zu analysierenden Probe anhand der folgenden Formel berechnet:

$$T = \frac{C/(M) - b}{a}$$

wobei a und b Konstanten sind, die die Gleichung y = ax + b einer Kalibrierungsgeraden des Verhältnisses zwischen dem zweiten Parameter und dem ersten Parameter als Funktion des Dieselkraftstoffanteils definieren.

**2.** Verfahren nach Anspruch 1, bei dem der zweite Parameter C für die Fläche mindestens eines mit einem $C_6$-$C_{25}$-Kohlenwasserstoff assoziierten Peaks des Chromatogramms repräsentativ ist.

**3.** Verfahren nach Anspruch 2, bei dem man eine Näherung des Dieselkraftstoffgehalts als Funktion eines zweiten Parameters C, der für die Fläche des mit $C_{20}$-Kohlenwasserstoff assoziierten Peaks des Chromatogramms repräsentativ ist, berechnet und dann als Funktion eines zweiten Parameters C, der für die Fläche von mehreren mit jeweils aus der Gruppe $C_{20}$ bis $C_{25}$ ausgewählten Kohlenwasserstoffen assoziierten Peaks repräsentativ ist, einen korrigierten Dieselkraftstoffgehalt berechnet, wobei die Zahl dieser Peaks von der berechneten Näherung abhängt.

**4.** Verfahren nach Anspruch 2 oder 3, bei dem der zweite Parameter für die Fläche von mehreren mit jeweils aus der Gruppe $C_{20}$ bis $C_{25}$ ausgewählten Kohlenwasserstoffen assoziierten Peaks repräsentativ ist.

**5.** Verfahren nach einem der vorhergehenden Ansprüche, bei dem man den ersten Parameter M anhand der folgenden Formel bestimmt:

$M = \alpha.M0/[C_5]$, wobei M0 die Fläche des mit dem $C_5$-Kohlenwasserstoff wie einem $C_5$-Alkan assoziierten Peaks ist, $\alpha$ ein Korrekturkoeffizient gegen eine Referenzkonzentration ist und $[C_5]$ die Konzentration an $C_5$-Kohlenwasserstoff in der Probe ist.

**6.** Verfahren nach einem der vorhergehenden Ansprüche, **dadurch gekennzeichnet, daß** die gebildete Mischung Kohlendisulfid in einem vorbestimmten Anteil enthält.

**7.** Verfahren nach einem der vorhergehenden Ansprüche mit einem vorhergehenden Schritt der Bestimmung der Gleichung der Kalibrierungsgerade, bei dem man:

- mehrere Probemischungen bildet, die ein Schmieröl und Dieselkraftstoff in vordefinierten verschiedenen Anteilen und $C_5$-Kohlenwasserstoff wie ein $C_5$-Alkan in einem vorbestimmten Anteil enthalten;
- für jede Probemischung:

- die Probemischung in den Injektor (11) eines Gaschromatographen (10) einspritzt;
- ein Chromatogramm der Probemischung erstellt;
- einen ersten Parameter M0 bestimmt, der für die Fläche eines mit dem $C_5$-Kohlenwasserstoff wie einem $C_5$-Alkan assoziierten Peaks des Chromatogramms repräsentativ ist;
- einen zweiten Parameter C0 bestimmt, der für die Fläche eines mit einem für Dieselkraftstoff repräsentativen Kohlenwasserstoff assoziierten Peaks des Chromatogramms repräsentativ ist;

- aus den für die verschiedenen Probemischungen erhaltenen Paaren von ersten und zweiten Parametern die Parameter a und b der Kalibrierungsgerade bestimmt.

**8.** Verfahren nach einem der vorhergehenden Ansprüche, bei dem man jedes Chromatogramm mit einem Flammenionisationsdetektor (13) erstellt und ein Rechenorgan die Integrationen des Chromatogramms zur Berechnung der Fläche jedes Peaks des Chromatogramms durchführt.

**Fig. 1**

**Fig. 2**

**Fig. 3**